# EUROPÄISCHE PATENTSCHRIFT

(11) **EP 2 634 579 B1**
(45) Veröffentlichungstag und Bekanntmachung des Hinweises auf die Patenterteilung: **17.12.2014**
(21) Anmeldenummer: 12001451.9
(22) Anmeldetag: 03.03.2012
(51) Int. Cl.: G01N 33/543

(54) **Verfahren zur Durchführung von Immuno-Assays in Schwerelosigkeit**
Method for performing immuno-assays in zero gravity
Procédé d'exécution de dosages immunologiques en apesanteur

(43) Veröffentlichungstag der Anmeldung: 04.09.2013
(73) Patentinhaber: Astrium GmbH, 82024 Taufkirchen (DE)
(72) Erfinder: Kern, Peter Dr., 88626 Salem (DE); Backes, Herbert, 66130 Saarbrücken (DE)
(74) Vertreter: Daub, Thomas

(56) Entgegenhaltungen:
- YOUNGEUN KWON ET AL: "Magnetic Bead Based Immunoassay for Autonomous Detection of Toxins", ANALYTICAL CHEMISTRY, Bd. 80, Nr. 22, 15. November 2008 (2008-11-15), Seiten 8416-8423, XP55033445, ISSN: 0003-2700, DOI: 10.1021/ac8010044
- MOSER Y ET AL: "Active superparamagnetic bead manipulation for immunoassays on-chip", TWENTIETH INTERNATIONAL CONFERENCE ON MINIATURIZED SYSTEMS FOR CHEMISTRY AND LIFE SCIENCES, 12. Oktober 2008 (2008-10-12), - 16. Oktober 2008 (2008-10-16), Seiten 1372-1374, XP002680448,
- RIDA A ET AL: "MANIPULATION OF SELF-ASSEMBLED STRUCTURES OF MAGNETIC BEADS FOR MICROFLUIDIC MIXING AND ASSAYING", ANALYTICAL CHEMISTRY, AMERICAN CHEMICAL SOCIETY, US, Bd. 76, Nr. 21, 1. November 2004 (2004-11-01), Seiten 6239-6246, XP001226101, ISSN: 0003-2700, DOI: 10.1021/AC049415J

## Beschreibung

Die Erfindung betrifft ein Verfahren zum kontrollierten Bewegen von magnetischen Trägern in einem Probenvolumen zur Durchführung von Immuno-Assays gemäß den Merkmalen des Patentanspruchs 1.

In der biochemischen Analytik ist der Einsatz von Immuno-Assays weit verbreitet. Diese Methode erlaubt die selektive quantitative oder qualitative Bestimmung von einzelnen (monoplex) oder mehreren (multiplex) Analyse-Parametern in einer überwiegend komplexen biologischen Matrix, wie z.B. Blut, Plasma, Serum, Urin, Speichel, Tränenflüssigkeit, Schweiß, Kulturmedien, Zellextrakten, Zellsuspensionen, usw.), welche eine Vielzahl von Stoffen enthalten kann.

Das generelle Prinzip von Immuno Assays ist, dass der gesuchte Analyt selektiv an einen spezifischen proteinbasierten Fängerantikörper oder an spezifische DNA, RNA oder darauf basierende funktionale Untergruppen oder Anschnitte (Capture Antibody = cAB) bindet und durch einen Detektionsantikörper (Detection Antibody = dAB) markiert wird. Der cAB befindet sich meist auf einem stationären Träger (feste Phase, solid phase).

In der Standardliteratur ist die Nomenklatur des Begriffs "Immuno Assay" uneinheitlich. Im Weiteren wird unter dem Begriff "Immuno Assay" sowohl für klassische Immuno Assays, wie auch ELISA (ELISA = Enzyme Linked ImmunoSorbent Assay) unter Verwendung von Enzymen verstanden:
a) Bei klassischen Immuno-Assays trägt der dAB trägt entweder einen Farbstoff oder ein Fluorophor, welche spektrometrisch oder fluorimetrisch nachgewiesen werden.
b) Eine weitere Immuno-Assay Variante sind die ELISAs (ELISA = Enzyme Linked ImmunoSorbent Assay). Diese verwenden ein an dAB gebundenes Enzyme als funktionales Labelelement. Die ELISAs, haben seit Anfang der 1980er Jahre, die RIAs (Radio-Immuno-Assays) ablösten, die als Label ein Radioisotop verwendeten. Das via den dAB an den Analyt-Antikörper-Komplex gebundene Enzym wandelt ein zugegebenes enzymspezifisches Substrat in eine nachweisbare Substanz um, welche spektrophotometrisch oder fluorimetrisch oder mittels eines anderen physikalischen Effekts, z.B.Chemilumineszenz in der Losung nachgewiesen werden kann.

Im terrestrischen Einsatz werden die verschiedenen Lösungen/Stoff sequentiell zugegeben. Die freien, nicht gebundenen Substanzen/Reaktanten werden durch Waschschritte entfernt. Die gebildeten Komplexe verbleiben durch ihre Bindung an die stationäre Phase im Reaktionsgefäß, wo sie dann detektiert werden können.

Eine spezielle Form der festen Phase sind mobile Träger. Dabei handelt es sich um so genannte Beads (Durchmesser: nm - mm, meist jedoch wenige µm), an deren Oberfläche die cAB-Moleküle gebunden sind. Nach dem Waschschritt werden diese Träger mittels Zentrifugation oder im Fall von magnetischen Trägern, mittels starker Magnete vom Überstand oder der Restlösung getrennt. Nach Abschluss der Gesamtreaktion des Immuno-Assays, werden die markierten Träger in den terrestrischen Anwendungen entweder in einem Durchflusszytometer, einem Lesegerät für Multiwellplatten oder einem Arrayreader ausgelesen. Das kann als integraler Messwert oder mittels Bildverarbeitung für jeden einzelnen Träger oder jeden Array-Spot erfolgen.

Die beschriebenen Schritte gelten für Immuno-Assays als Sandwich-Assay, als kompetitiver Assay oder auch in der Form eines ELISAs.

Immuno-Assays sollen auch bei Weltraum-Flügen unter reduzierter Schwerkraft, bis hin zu Schwerelosigkeit (µg) zum Einsatz kommen. Das bedeutet, dass der Stofftransport oder die Stofftrennung durch die reduzierte oder fehlende Schwerkraft verlangsamt oder gar behindert sind. Während der Probenvorbereitung werden die Reaktionspartner auf der Erde in speziellen mechanischen Mischern (z.B. Orbital-Mischem oder Orbital-Shaker) bewegt. Das Absetzen zur Beobachtung erfolgt mittels Schwerkraft.

Immuno-Assays mit magnetischen Trägern sind für Anwendung auf der Erde unter 1 µg weit verbreitet. Bislang wurden die magnetischen Trägern jedoch primär zur Abtrennung während eines Waschschritts verwendet. Die terrestrische Prozessführungen von Immuno-Assays, zur Zellkonzentration oder Abtrennung sind für Weltraumanwendung nicht geeignet.

Aufgabe der Erfindung ist es, ein Verfahren anzugeben, mit welchem die Durchführung von Immuno-Assays mit magnetischen Trägern unter Schwerelosigkeit oder reduzierter Schwerkraft möglich ist.

Diese Aufgabe wird durch das Verfahren gemäß den Merkmalen des geltenden Anspruchs 1 gelöst. Vorteilhafte Ausgestaltungen der Erfindung sind Gegenstand von Unteransprüchen.

Gemäß der Erfindung werden zum kontrollierten Bewegen von magnetischen Trägern in einem Probenvolumen zur Durchführung von Immuno-Assays in Schwerelosigkeit, die magnetischen Träger innerhalb des Probenvolumens mittels bezüglich wenigstens einer Raumachse des Probenvolumens verschiebbar angeordneter Permanentmagnete bewegt.

Der Einsatz von magnetischen Trägern z.B. als feste Phase ermöglicht aktives, kontrolliertes, konvektives Mischen der Reaktionspartner, durch äußere Magnetfelder, die z.B. sequentiell aus verschiedenen Richtungen wirken. Zudem wird der Stofftransport verbessert und die Reaktionsgeschwindigkeit erhöht. Ein weiterer Vorteil ist, dass die Prozessführung in Schwerelosigkeit reproduzierbar wird. Schließlich ist ein flächiges Positionieren der magnetischen Träger zum Zweck der Detektion möglich (z.B. im Fokus eines Mikroskops), durch ein gezielt aktivierbares, gerichtetes Magnetfeld zu einem vorgegebenen Zeitpunkt.

Darüber hinaus ist es möglich die magnetischen Träger in einem bestimmten Bereich, z. B. während eines Fluidwechsels oder einem Waschvorgang durch ein gezielt aktivierbares, gerichtetes Magnetfeld zu sammeln oder festzuhalten.

Zusätzlich können die magnetischen Träger, die mit einem cAB belegt sind auch zum Binden an spezifische Zelltypen oder Membranrezeptoren verwenden werden, und diese bei Weltraumexperimenten mit reduzierter Schwerkraft, abgetrennt oder aufkonzentriert oder durch mechanisches Verschieben der Detektion zugeführt werden.

Die fehlende oder reduzierte Schwerkraft wird beim Einsatz von Immuno-Assays im Weltraum durch den konsequente Einsatz von magnetischen Trägern kompensiert. Die magnetischen Träger werden je nach Prozessschritt durch externe, kontrolliert aktivierte Magnetfelder beeinflusst.

Zum Mischen von magnetischen Trägern in einem Probenvolumen werden vorteilhaft die Permanentmagnete bezüglich des Probenvolumens diametral gegenüber angeordnet.

Zum Positionieren von magnetischen Trägern auf einer Ebene innerhalb des Probenvolumens werden vorteilhaft Permantmagnete auf einer zur Ebene senkrechten Raumachse, wobei die Permantmagnete bezüglich der Ebene diametral den zu positionierenden magnetischen Trägern gegenüber liegen, in einem ersten Schritt in Richtung des Probenvolumens bewegt und in einem zweiten Schritt vom Probenvolumen entfernt.

Die Erfindung sowie weitere vorteilhafte Ausgestaltungen der Erfindung werden im Weiteren anhand von Figuren näher erläutert. Es zeigen:
- Fig. 1: eine beispielhafte schematische Anordnung zur Durchführung des erfindungsgemäßen Verfahrens in einer ersten Anwendung,
- Fig. 2: eine beispielhafte schematische Anordnung zur Durchführung des erfindungsgemäßen Verfahrens in einer zweiten Anwendung.
- Fig. 3: eine beispielhafte Ausführung eines Permanentmagneten.

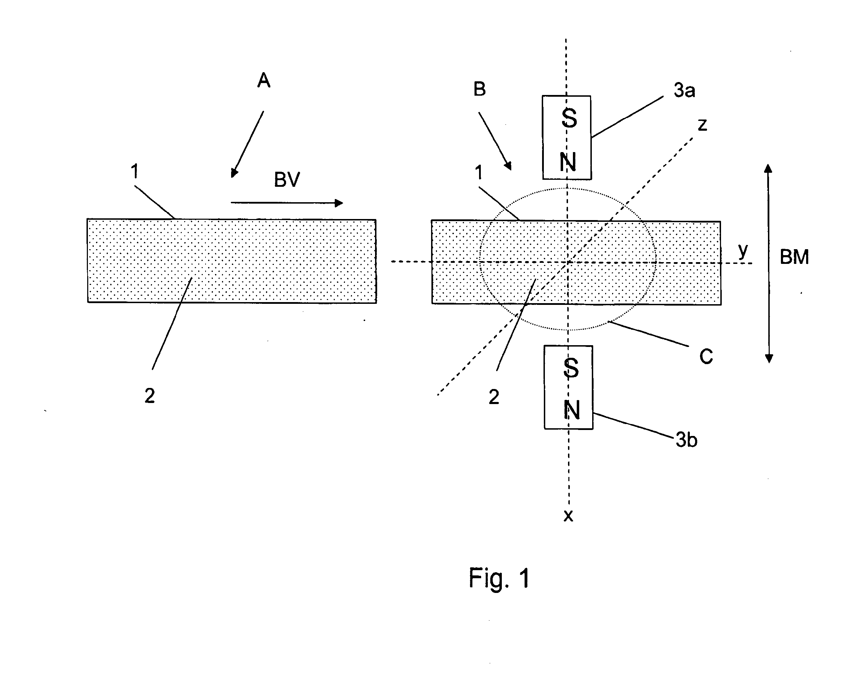

Fig. 1 zeigt eine beispielhafte schematische Anordnung zum Mischen von magnetischen Trägern 2 innerhalb eines Probenvolumens 1. Außerhalb des Probenvolumens 1 sind Permanentmagnete 3a, 3b auf einer Raumachse x,y,z des Probenvolumens 1 angeordnet. Zur übersichtlicheren Darstellung sind in Fig. 1 lediglich 2 Permanentmagnete 3 auf der Raumachse x dargestellt. Selbstverständlich können weitere Permanentmagnete 3a, 3b auf den weiteren Raumachsen y, z angeordnet werden.

Die beiden Permanentmagnete 3a, 3b sind bezüglich des Probenvolumens 1 diametral gegenüber angeordnet, d.h. in einen Bereich C zwischen den beiden Permanentmagneten 3a, 3b ist das Probenvolumens 1 einbringbar. Jeder Permanentmagnet 3a, 3b besteht bekanntermaßen aus einem Nordpol N und einem Südpol S. Zweckmäßig sind die beiden Permanentmagnet 3a, 3b so angeordnet, dass sich jeweils Nord- und Südpol gegenüberliegen.

Fig. 1 zeigt die Anordnung mit dem Probenvolumen 1 in einer ersten Position A, in welcher sich das Probenvolumen 1 außerhalb des Bereiches B zwischen den beiden Permanentmagneten 3a, 3b befindet. Das Probenvolumen 1 kann entsprechend der Pfeilrichtung BV in eine Position B verschoben werden, so dass sich das Probenvolumen 1 im Bereich C befindet. Selbstverständlich ist es auch möglich, dass die beiden Permanentmagnete 3a, 3b entsprechend verschoben werden.

Zum Mischen der magnetischen Trägern 2 in dem Probenvolumen 1 wird das Probenvolumen 1 in Position B gebracht. Anschließend werden die beiden Permanentmagnete 3a, 3b gleichphasig entsprechend der Pfeilrichtung BM hin- und herbewegt. Die magnetischen Träger 2 werden im Probenvolumen 1 nun abwechselnd entsprechend des anliegenden Magnetfeldes ausgerichtet und entsprechend bewegt. Durch die gleichphasige Hin- und Herbewegung der beiden Permanentmagnete 3a, 3b wird eine Durchmischung der magnetischen Träger 2 im Probenvolumen 1 bewirkt.

Durch entsprechende Anordnung und Bewegung von weiteren Permanentmagneten auf den Raumachsen y, z kann die Durchmischung verbessert werden.

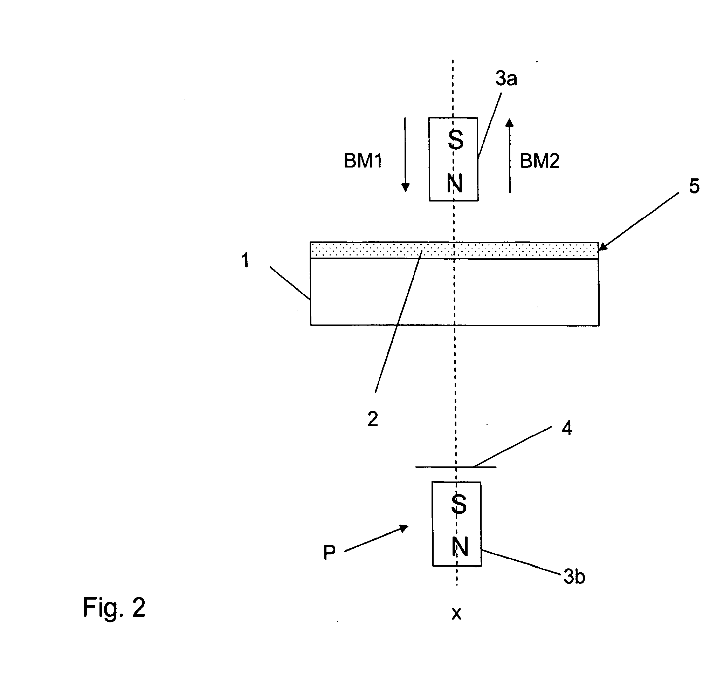

Fig. 2 zeigt eine beispielhafte schematische Anordnung zum Positionieren von magnetischen Trägern 2 innerhalb eines Probenvolumens 1. Die Darstellung zeigt ein Probenvolumen in Position B entsprechend Fig. 1. Zur Positionierung von magnetischen Trägern 2 auf der Ebene 5 wird der im weiteren als Positionierungspermanentmagnet bezeichneter Permanentmagnet 3a verwendet, welcher auf einer zur Positionierungsebene 5 senkrechten Achse x angeordnet wird. Dieser Permanentmagnet 3a welcher bezüglich der Positionierungsebene den zu positionierenden magnetischen Trägern 2 diametral gegenüber liegt kann entsprechend der Pfeilrichtungen BM1, BM2 verschoben werden.

Ein anderer bezüglich des Probenvolumens 1 diametral zu dem Positionierungspermanentmagnet 3a auf der Raumachse x angeordnete Permanentmagnet 3b wird in eine Parkposition P verschoben und mittels einer Abschirmvorrichtung 4 geschützt, so dass Magnetfelder des Permanentmagnet 3b keinen Einfluss auf die magnetischen Träger 2 im Probenvolumen 1 nehmen können.

Zur Positionierung der magnetischen Träger 2 im Probenvolumen 1 wird der Positionierungspermanentmagnet 3a in Richtung BM1 der Ebene 5 verschoben. Dadurch werden die magnetischen Träger 2 in Richtung der Ebene 5 ausgerichtet und bewegt. Anschließend wird der Positionierungspermanentmagnet 3a in Richtung BM2 verschoben und in eine entsprechende Parkposition P (nicht dargestellt) verschoben.

Bei der Weltraumanwendung verbleiben die magnetischen Träger bis zum Ende der Detektion in dieser Position, da wegen der reduzierten Schwerkraft keine Sedimentation oder Thermalkonvektion im Probevolumen stattfindet

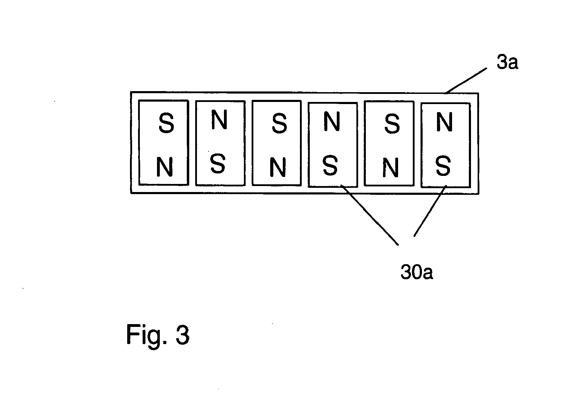

Fig. 3 zeigt beispielhaft die Ausführung eines Permanentmagneten. Vorteilhaft sind die Permanentmagnete als Matrix ausgeführt. Der Permanentmagnet 3a umfasst mehrere Permanentmagnete 30a, welche zweckmäßig als Matrix angeordnet sind, wobei die Permanentmagnete 30a alternierend angeordnet werden.

## Patentansprüche

1. Verfahren zum kontrollierten Bewegen von magnetischen Trägern (2) in einem Probenvolumen (1) zur Durchführung von Immuno-Assays in Schwerelosigkeit oder reduzierter Schwerkraft, wobei wegen der reduzierten Schwerkraft keine Sedimentation oder Thermalkonvektion im Probenvolumen (1) stattfindet und wobei die magnetischen Träger (2) innerhalb des Probenvolumens (1) mittels bezüglich wenigstens einer Raumachse (x, y, z) des Probenvolumens (1) verschiebbar angeordneter Permanentmagnete (3a, 3b) bewegt werden,
**dadurch gekennzeichnet, dass**
durch den Einsatz von magnetischen Trägern (2) aktives, kontrolliertes, konvektives Mischen von Reaktionspartnern durch äußere Magnetfelder bewirkt wird, wobei die magnetischen Träger (2) während eines Fluidwechsels oder eines Waschvorgangs durch ein gezielt aktivierbares, gerichtetes Magnetfeld gesammelt oder festgehalten werden und
wobei die magnetischen Träger (2) flächig zum Zweck der Detektion, beispielsweise im Fokus eines Mikroskops, durch ein gezielt aktivierbares, gerichtetes Magnetfeld positioniert werden.

2. Verfahren nach Anspruch 1,
**dadurch gekennzeichnet, dass**
die Permanentmagnete (3a, 3b) bezüglich des Probenvolumens (1) diametral gegenüber angeordnet werden.

3. Verfahren nach Anspruch 1 oder 2,
**dadurch gekennzeichnet, dass**
zum Mischen der magnetischen Träger die auf einer Raumachse (x, y, z) angeordneten Permanentmagnete (3a, 3b) gleichphasig bewegt werden.

4. Verfahren nach einem der vorhergehenden Ansprüche,
**dadurch gekennzeichnet, dass**
zum Positionieren von magnetischen Trägern auf einer Ebene (5) innerhalb des Probenvolumens (1) Permanentmagnete (3a, 3b) auf einer zur Ebene (5) senkrechten Raumachse (x, y, z), wobei die Permanentmagnete (3a, 3b) bezüglich der Ebene (5) diametral den zu positionierenden magnetischen Trägern (2) gegenüber liegen, in einem ersten Schritt in Richtung (BM1) des Probenvolumens (1) bewegt werden und in einem zweiten Schritt vom Probenvolumen (1) bewegt (BM2) werden.

5. Verfahren nach einem der vorangehenden Ansprüche,
**dadurch gekennzeichnet, dass**
die Permanentmagnete (3a, 3b) bei Nichtbenutzung in einem Verfahrensschritt in eine abgeschirmte Parkposition (P) gebracht werden.

6. Verfahren nach einem der vorangehenden Ansprüche,
**dadurch gekennzeichnet, dass**
die Permanentmagnete (3a, 3b) jeweils in Form eines Arrays angeordnet werden.

## Claims

1. Method for controlled movement of magnetic carriers (2) in a sample volume (1) for performing immunoassays under weightless conditions or reduced gravity, wherein because of the reduced gravity no sedimentation or thermal convection occurs in the sample volume (1) and wherein the magnetic carriers (2) are moved within the sample volume (1) by means of permanent magnets (3a and 3b) movably arranged relative to at least one spatial axis (x, y, z) of the sample volume (1),
**characterized in that**
by the use of magnetic carriers (2) active, controlled, convective mixing of reaction partners is effected by external magnetic fields, wherein during a change of fluids or a washing process the magnetic carriers (2) are collected or arrested by a directional magnetic field which can be specifically activated, and wherein the magnetic carriers (2), for the purpose of detection, for example in the focus of a microscope, are positioned in a plane by a directional magnetic field which can be specifically activated.

2. Method according to Claim 1,
**characterized in that**
the permanent magnets (3a and 3b) are arranged diametrically opposite relative to the sample volume (1).

3. Method according to Claim 1 or 2,
**characterized in that**
for mixing of the magnetic carriers the permanent magnets (3a and 3b) arranged on a spatial axis (x, y, z) are moved in phase.

4. Method according to one of the preceding claims,
**characterized in that**
for positioning of magnetic carriers in a plane (5) within the sample volume (1) permanent magnets (3a and 3b) are moved on a spatial axis (x, y, z) that is perpendicular to the plane (5) in a first step in a direction (BM1) of the sample volume (1) and in a second step away (BM2) from the sample volume (1), wherein the permanent magnets (3a, 3b) are situated, relative to the plane (5), diametrically opposite the magnetic carriers (2) to be positioned.

5. Method according to one of the preceding claims,
**characterized in that**
when not used in a process step, the permanent magnets (3a and 3b) are brought into a shielded parking position (P).

6. Method according to one of the preceding claims,
**characterized in that**
the permanent magnets (3a and 3b) are each arranged in the form of an array.

## Revendications

1. Procédé pour déplacer de manière contrôlé des supports magnétiques (2) dans un volume échantillon (1), prévu pour réaliser des dosages immunologiques en apesanteur ou sous gravité réduite,
la gravité réduite empêchant qu'une sédimentation ou d'une convection thermique aient lieu dans le volume échantillon (1),
et les supports magnétiques (2) étant déplacés à l'intérieur du volume échantillon (1) au moyen d'aimants permanents (3a, 3b) disposés de manière deplaçable par rapport à au moins un axe spatial (x, y, z) du volume échantillon(1), **caractérisé en ce que**
par l'emploi des supports magnétiques un mélangement actif, contrôlé et convectif de partenaires de réaction est effectué sous l'action de champs magnétiques extérieurs,
les supports magnétiques (2) étant, pendant un changement de fluide ou une opération de lavage, rassemblés ou détenus par un camp magnétique orienté qui est spécifiquement activable, et les supports magnétiques (2) étant positionnés dans un plan dans le but de la détection, par exemple au foyer d'un microscopes, par un champ magnétique orienté qui est spécifiquement activable.

2. Procédé selon la revendication 1,
**caractérisé en ce que**
les aimants permanents (3a, 3b) sont disposés en positions diamétralement opposées par rapport au volume échantillon(1).

3. Procédé selon les revendications 1 ou 2,
**caractérisé en ce que**
pour le mélangement des supports magnétiques, les aimants permanents (3a, 3b) disposés sur un axe spatial (x, y, z) sont déplacés à une même phase.

4. Procédé selon l'une quelconque des revendications précédentes,
**caractérisé en ce que**
pour positionner des supports magnétiques sur un plan (5) à l'intérieur du volume échantillon (1), des aimants permanents (3a, 3b) sont déplacés sur un axe spatial (x, y, z) perpendiculaire au plan (5) dans une première étape en direction (BM1) du volume échantillon (1), et sont déplacés (BM2) dans une deuxième étape du volume échantillon (1), les aimants permanents (3a, 3b) étant situés, par rapport au plan (5), diamétralement opposées aux supports magnétiques (2) à positionner.

5. Procédé selon l'une quelconque des revendications précédentes,
**caractérisé en ce**
**que** lorsqu'ils ne sont pas utilisés, les aimants permanents (3a, 3b) sont amenés dans une position de stationnement (P) blindée dans une étape du procédé.

6. Procédé selon l'une quelconque des revendications précédentes,
**caractérisé en ce que**
les aimants permanents (3a, 3b) sont respectivement disposés en batterie.
